# DEMANDE DE BREVET EUROPEEN

(11) **EP 1 491 507 A1**
(43) Date de publication de la demande: **29.12.2004**
(21) Numéro de dépôt: 04291296.4
(22) Date de dépôt: 21.05.2004
(51) Int. Cl.: C02F 11/04

(54) **Procédé de digestion anaérobie de boues et digesteur**

(30) Priorité: 24.06.2003 FR 0307631
(71) Demandeur: Ergalia, 75015 Paris (FR)
(72) Inventeur: Philip, Hervé, 34660 Cournonsec (FR); Maunoir, Siegfried, 34560 Montbazin (FR)
(74) Mandataire: Derambure, Christian

(57) **Abrégé**

Le procédé consiste à :
- introduire dans la partie amont du digesteur (200) un flux de boues fraîches de concentration en matière sèche inférieure à 40 g/l ;
- maintenir dans le digesteur une couche de boues de faible épaisseur ;
- faire circuler les effluents au-dessus et à travers la couche de boues, sensiblement horizontalement, et évacuer les effluents hors du digesteur, en même temps que les produits solubles de la digestion des boues.

La cuve (201) du digesteur est divisée en compartiments (218a - 218d) par des parois (213a - 213c) transversales à l'écoulement des effluents munies d'une ouverture de communication (216) en partie inférieure.

## Description

L'invention concerne un procédé de digestion anaérobie de boues issues du traitement des effluents usés, un digesteur anaérobie de boues fraîches contenant des effluents, ainsi qu'une station d'épuration comprenant notamment un tel digesteur.

La digestion anaérobie de boues issues du traitement des effluents usés, par exemple les effluents domestiques, consiste en une hydrolyse et une fermentation méthanique ayant un important pouvoir de destruction cellulaire, de sorte à pouvoir éliminer une quantité non négligeable des matières organiques contenues dans ces boues.

Les boues sont par exemple issues de la décantation des effluents bruts. En sortie du digesteur, on obtient d'une part des boues digérées, et d'autre part des effluents, ces deux fractions pouvant ensuite subir séparément un certain nombre de traitements supplémentaires.

On connaît du document US 6 673 243 un digesteur anaérobie de boues comprenant une cuve divisée en plusieurs compartiments successifs au moyens de parois transversales ne s'étendant pas jusqu'en haut de la cuve. Dans le premier compartiment amont, en partie inférieure, les boues à traiter sont introduites par un conduit d'alimentation, les effluents traités étant évacués depuis le dernier compartiment aval par un conduit d'évacuation.

La paroi transversale médiane comporte une ouverture en partie inférieure, permettant le passage des boues et des effluents du compartiment amont au compartiment aval qu'elle définit.

En revanche, les autres parois transversales sont dépourvues d'ouverture inférieure. Il s'ensuit que, d'une part, l'écoulement des effluents est alternativement ascendant puis descendant, et, d'autre part, les boues peuvent s'accumuler dans un compartiment sans pouvoir, une fois digérées, s'écouler vers l'aval. L'efficacité du digesteur s'en trouve considérablement diminuée.

L'invention a pour but de résoudre ces problèmes.

A cet effet, et selon un premier aspect, l'invention concerne un procédé de digestion anaérobie de boues, caractérisé en ce qu'il comprend les étapes consistant à :
- introduire dans la partie amont d'un digesteur anaérobie de boues un flux de boues fraîches, lesdites boues fraîches contenant des effluents et présentant une concentration en matière sèche inférieure à 40 g/l ;
- maintenir dans le digesteur une couche de boues d'épaisseur inférieure à 0,6m, les boues étant transformées par digestion anaérobie ;
- faire circuler lesdits effluents dans le digesteur, au-dessus et à travers la couche de boues, de façon sensiblement horizontale au moins dans la partie utile du digesteur, de la partie amont vers la partie aval du digesteur, et évacuer lesdits effluents hors du digesteur, de sorte à évacuer les produits solubles de la digestion des boues hors du digesteur ;
- évacuer les boues hors du digesteur.

Ainsi, dès le début de la digestion par hydrolyse des matières sèches, les composants solubles formés sont extraits de la couche de boues. De la sorte sont évités ou considérablement atténués les phénomènes d'inhibition, ou du moins de ralentissement de la digestion des boues dans la cuve. La digestion continue donc à s'effectuer à une vitesse optimisée, et le temps de séjour nécessaire des boues dans le digesteur est fortement diminuée.

Selon une réalisation possible, on maintient dans le digesteur une couche de boues d'une épaisseur inférieure à 0,5 m. Par exemple, les boues fraîches présentent une concentration en matière sèche inférieure à 20 g/l.

Selon un deuxième aspect, l'invention concerne un digesteur anaérobie de boues fraîches contenant des effluents, pour la mise en oeuvre du procédé tel que précédemment décrit.

Le digesteur comprend une cuve ayant un fond sensiblement horizontal, un conduit d'alimentation de la cuve en boues fraîches, un conduit d'évacuation des effluents hors de la cuve, et des moyens d'évacuation des boues digérées hors de la cuve, ladite cuve comportant au moins une paroi transversale à l'écoulement des effluents, la paroi définissant un compartiment amont et un compartiment aval, de sorte que la cuve présente un premier compartiment amont, dans lequel débouche le conduit d'alimentation en boues fraîches, et un dernier compartiment aval, dans lequel débouche le conduit d'évacuation des effluents.

Selon l'invention, la paroi présente, à sa partie inférieure, une ouverture de communication du compartiment amont au compartiment aval, de sorte à permettre le passage des boues et la circulation des effluents, au-dessus et à travers la couche de boues maintenue au fond de la cuve, de façon sensiblement horizontale du premier compartiment amont au dernier compartiment aval.

La paroi transversale s'étend depuis le fond de la cuve sur une hauteur inférieure à la hauteur de la cuve, de sorte à permettre le débordement des matières flottantes dans les effluents d'un compartiment à un autre.

Par exemple, le digesteur comporte au moins deux parois transversales présentant chacune, à leur partie inférieure, une ouverture de communication, de sorte à définir au moins trois compartiments successifs dans la cuve.

Enfin, selon un troisième aspect, l'invention concerne une station d'épuration d'effluents usés comprenant au moins un tel digesteur.

Les autres caractéristiques de l'invention résultent de la description qui suit de modes de réalisation, description effectuée en référence aux figures annexées dans lesquelles :
- la figure 1 est une vue schématique en coupe, selon un plan vertical longitudinal médian, d'un digesteur selon l'invention ;
- la figure 2 est une vue schématique en perspective du digesteur de la figure 17, vu selon la même coupe ;
- la figure 3 est une représentation schématique d'une station d'épuration selon l'invention, comprenant notamment un décanteur, un filtre ou une installation de filtration, et un digesteur anaérobie de boues, illustrant en outre les étapes d'un procédé de traitement des effluents usés dans ladite station d'épuration ;
- la figure 4 est une vue schématique en section transversale d'un décanteur prévu dans la station d'épuration, selon un mode de réalisation possible ;
- la figure 5 est une vue en coupe, selon la ligne AA, du décanteur de la figure 4 ;
- la figure 6 est une vue en coupe du décanteur de la figure 4, selon la ligne BB de la figure 5 ;
- la figure 7 est une vue schématique en coupe verticale longitudinale d'un filtre prévu dans la station d'épuration, selon un mode de réalisation possible.

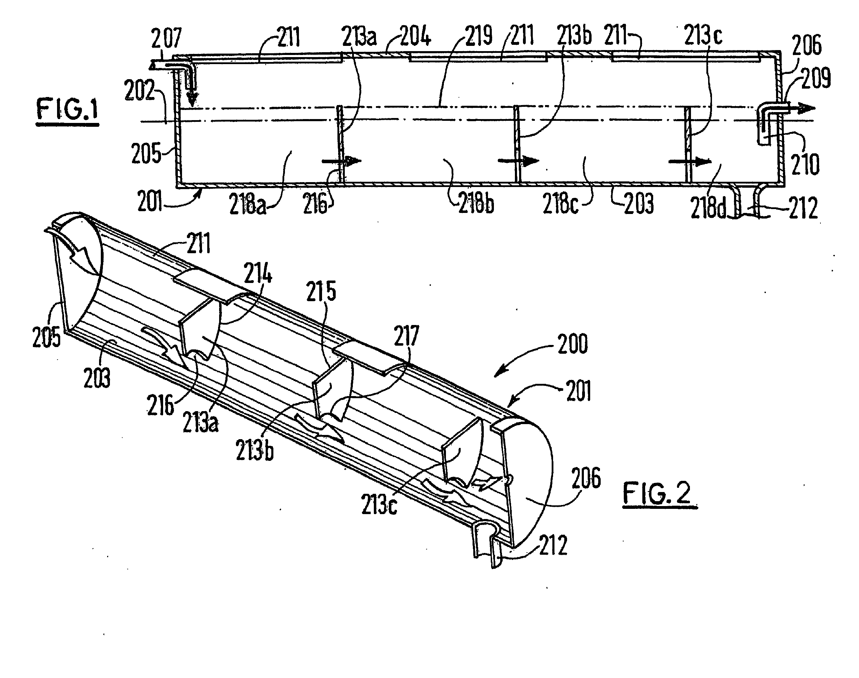

On se réfère tout d'abord aux figures 1 et 2 qui représentent un digesteur 200 selon l'invention, destiné au traitement de boues fraîches contenant des effluents. Ces boues sont issues d'un traitement préalable d'effluents usés, par exemple issues d'une décantation.

Le digesteur 200 comprend une cuve 201 d'axe 202, par rapport auquel est défini le terme « longitudinal ». La description qui suit est effectuée dans le cas d'une cuve 201 de forme générale sensiblement cylindrique. Toutefois, la cuve peut présenter une section verticale sensiblement rectangulaire : on peut alors prévoir une cuve très large (dans un plan horizontal) tout en conservant une hauteur réduite.

La cuve 201 est fermée, à l'exception de trois ouvertures principales et d'une ou plusieurs trappes de visite, comme on le verra ci-après, de sorte à assurer l'absence d'oxygène pour permettre la fermentation méthanique. La cuve 201 présente une hauteur relativement faible (correspondant au diamètre du cylindre), de l'ordre de 50 à 70 cm, et une longueur comprise entre 2 et 3 m. A titre d'exemple, la cuve peut avoir un volume de 250 à 300 I, par exemple 270 I.

Dans le cas d'une cuve de section rectangulaire, le volume est augmenté en fonction de la largeur, et peut atteindre plusieurs milliers de litres.

La cuve 201 est positionnée de sorte que l'axe 202 soit sensiblement horizontal. Elle comporte une paroi latérale sensiblement cylindrique définissant un fond 203 et une paroi supérieure 204, ainsi qu'une une paroi d'extrémité amont 205 et une paroi d'extrémité aval 206, sensiblement en forme de disque.

En partie supérieure de la paroi amont 205 est ménagée une ouverture permettant l'entrée dans le digesteur 200 des boues fraîches, par un conduit d'alimentation 207. Le conduit d'alimentation 207 se prolonge à l'intérieur de la cuve 201 sensiblement parallèlement à l'axe 202 sur une faible longueur, puis verticalement vers le bas sur une longueur de l'ordre de 10 cm par exemple, de sorte à former à l'intérieur de la cuve 201, et à proximité de la paroi amont 205, un coude 208 ouvert vers le fond 203 de la cuve 201. Le conduit d'alimentation 207 peut déboucher au-dessus ou en-dessous du niveau d'effluents présent dans la cuve 201.

La paroi aval 206 présente, sensiblement à mi hauteur, une ouverture permettant l'évacuation des effluents hors du digesteur 200, par un conduit d'évacuation 209.

Le conduit d'évacuation 209 se prolonge à l'intérieur de la cuve 201 sensiblement parallèlement à l'axe 202 sur une faible longueur, puis verticalement vers le bas sur une longueur de l'ordre de 10 cm par exemple, de sorte à former à l'intérieur de la cuve 201, et à proximité de la paroi aval 206, un coude 210 ouvert vers le fond 203 de la cuve 201, formant une prise siphoïque. La prise siphoïque se situe sensiblement à mi hauteur entre le fond 203 de la cuve 21 et le niveau d'effluents maintenu dans la cuve 201, de sorte à ne laisser échapper ni les matières flottantes, ni les matières reposant sur le fond 203.

La paroi supérieure 204 de la cuve 201 peut quant à elle comporter au moins une trappe de visite 211, et trois trappes de visite dans le mode de réalisation représenté. A la paroi supérieure 204 peuvent également être raccordés des moyens de prélèvement du gaz produits par une digestion méthanique.

Enfin, dans le fond 203 de la cuve 201, au voisinage de la paroi aval 206, est ménagé un orifice d'évacuation des boues digérées, ledit orifice étant connecté à un conduit d'évacuation 212 desdites boues, des moyens de coupure dudit conduit d'évacuation 212 étant prévus.

Le digesteur 200 comporte en outre trois parois transversales respectivement amont 213a, intermédiaire 213b, et aval 213c. Selon d'autres réalisations envisageables, le digesteur pourrait comprendre un nombre différent de parois transversales, par exemple compris entre 1 et 6, selon les besoins.

Les parois 213a, b, c présentent sensiblement la même forme, à savoir celle d'une portion de disque. Le contour annulaire 214 des parois épouse la forme intérieure de la cuve 201, depuis le fond 203 et jusqu'à une hauteur inférieure à la hauteur de la cuve, de sorte que chaque paroi 213a, b, c s'étend sur toute la largeur de la cuve 201. Les parois 213a, b, c présentent par ailleurs un bord supérieur 215 sensiblement plan et horizontal, situé à une distance du fond 203 de la cuve 201 comprise entre 40 et 70% de la hauteur de la cuve 201. Par exemple, la hauteur des parois est de l'ordre de 20 à 40 cm, notamment 30 cm.

Dans la partie inférieure de chaque paroi 213a, b, c est ménagée une ouverture de communication 216, sensiblement en forme de disque. L'ouverture 216 comprend un bord supérieur 217 sensiblement annulaire et s'étend depuis le fond 203 de la cuve 201 sur une hauteur comprise entre 30 et 60% de la hauteur de la paroi transversale 213a, b, c. Par exemple, l'ouverture 216 s'étend sur une hauteur de l'ordre de 10 à 15 cm.

Chaque paroi transversale 213a, b, c définit un compartiment amont et un compartiment aval, de sorte que la cuve 201 du digesteur 200 est divisée longitudinalement en quatre compartiments 218a, 218b, 218c, 218d :
- un premier compartiment amont 218a, dans lequel débouche le conduit d'alimentation 207 en boues fraîches, par exemple en partie supérieure ;
- deux compartiments intermédiaires successifs 218b, 218c ;
- un dernier compartiment aval 218d, dans lequel débouche le conduit d'évacuation 209 des effluents.
   Les parois 213a, b, c permettent de retenir dans chaque compartiment 218a à 218d les boues formées par agglomération des matières en suspension, tandis que les matières flottantes en excès peuvent passer par débordement d'un compartiment amont à un compartiment aval, voire l'inverse.

Les compartiments peuvent présenter des longueurs axiales similaires ou, en variante, différentes. Dans l'exemple représenté, les trois premiers compartiments 218a, b, c ont des longueurs sensiblement identiques, tandis que le dernier compartiment aval 218d présente une longueur moindre, voisine de la moitié de celle des autres compartiments.

Le nombre de compartiments et la longueur du digesteur peuvent être diminués, ou au contraire augmentés pour affiner le traitement.

On décrit à présent le mode de fonctionnement du digesteur 200.

Les boues fraîches contenant des effluents sont introduites dans la cuve 201 du digesteur 200 par le conduit d'alimentation 207.

Par « boues fraîches » on entend les boues ayant une concentration en matière sèche relativement faible, inférieure à 25 g/l, voire inférieure à 20 g/l. On peut notamment introduire dans le digesteur des boues ayant une concentration comprise entre 5 et 10 g/l. Une concentration plus faible est possible, mais le procédé est alors moins économique (plus grand volume de digesteur, débits de pompage plus importants, etc.).

Ces boues peuvent être issues d'une décantation, et donc contenir naturellement encore des effluents. En variante, il peut s'agir de boues préalablement épaissies (concentration en matières sèches de l'ordre de 10 à 15 %), notamment pour faciliter leur transport d'un site de traitement à un autre.

Dans ce cas, les boues épaissies sont diluées avec des effluents de dilution-par exemple des effluents décantés issus de la décantation d'effluents usés-avant d'être introduites dans le digesteur, de façon à obtenir une concentration en matières sèches compatible avec le procédé.

Les boues se déposent sur le fond 203 de la cuve 201, sous forme d'une couche maintenue de faible épaisseur, par exemple inférieure à 0,5 m, voire inférieure à 0,3 m. Selon une réalisation, l'épaisseur de la couche de boues peut être inférieure à 0,2 m. Il est maintenu dans la cuve 201 un niveau 219 d'effluents, comme représenté sur les figures 1 et 2. Le niveau 219 d'effluents affleure sensiblement l'extrémité supérieure du conduit d'évacuation 209 et le bord supérieur 215 des parois 213a, b, c.

Les boues sont progressivement dégradées dans le digesteur 200. Au fur et à mesure de leur digestion, les boues se liquéfient et passent dans les compartiments successifs 218a à 218d, par les ouvertures de communication 216 ménagées dans les parois transversales 213a à c.

Quant aux effluents, ils s'écoulent sensiblement parallèlement à l'axe 202, du moins dans la partie utile de la cuve 201, c'est-à-dire à l'exception des zones d'alimentation et d'évacuation, où les effluents s'écoulent localement de façon descendante depuis le conduit d'alimentation 207 vers le fond 203 de la cuve, respectivement ascendante depuis le fond 203 de la cuve vers le conduit d'évacuation 209.

Les effluents circulent ainsi sensiblement horizontalement, à travers les ouvertures 216, entre le fond 203 de la cuve 201 et le bord supérieur 217 desdites ouvertures 216, et ce depuis le premier compartiment amont 218a jusqu'au dernier compartiment aval 218d. Lors de cet écoulement, les effluents circulent au-dessus et à travers la couche de boues. De ce fait, d'une part, ils emportent avec eux les produits solubles issus de la digestion des boues. Et, d'autre part, ils forcent les boues à se déplacer vers les compartiments plus en aval.

Les effluents et les produits solubles sont ensuite évacués hors de la cuve 201 par le conduit d'évacuation 209 et acheminés, le cas échéant, vers une autre unité de traitement.

Les boues digérées contenues dans le dernier compartiment aval 218d sont évacuées par le conduit d'évacuation 212, par simple gravité ou à l'aide de moyens de pompage appropriés. Les vidanges peuvent être partielles ou totales, et régulières (vidange périodique du digesteur), ou consister en des sous-tirages périodiques ou continus, quotidiens par exemple. En variante, la cuve 201 est dépourvue de conduit d'évacuation des boues, les boues étant alors temporairement stockées dans le digesteur 200 et périodiquement évacuées via les trappes 211.

Le digesteur selon l'invention permet d'éliminer par solubilisation ou gazéification un minimum de 40% des matières sèches des boues, et jusqu'à plus de 90 %, et ce avec un temps de séjour des boues dans le digesteur compris entre 5 et 15 jours, et sans nécessiter de chauffage du digesteur, pour autant que sa température reste supérieure à 10°C. Avec un chauffage du digesteur (par exemple à une température comprise entre 20 et 35°C), des temps de séjour plus courts sont possibles, la performance du digesteur étant améliorée.

Dans ces conditions, l'épaississement des boues digérées est largement facilitée, puisqu'il ne reste que 50 % à moins de 10 % de boues.

Ceci est à comparer aux procédés de l'art antérieur, dans lesquels les boues sont épaissies préalablement à leur entrée dans le digesteur. Leur temps de séjour, sous forme de couche relativement épaisse, est généralement compris entre 12 et 40 jours, le digesteur devant être chauffé entre 25 et 35°C, et ce pour obtenir une élimination d'environ 30% des matières sèches des boues.

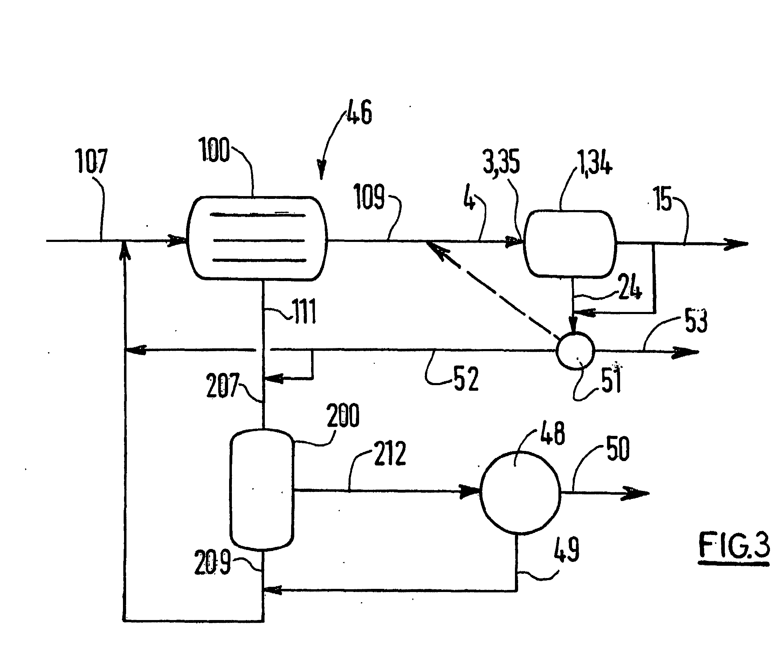

On décrit à présent, en référence à la figure 3, une station d'épuration 46 des effluents usés.

Les effluents bruts sont amenés via un conduit d'alimentation 107 en entrée d'un décanteur primaire 100, de sorte à être débarrassés d'une part importante de leurs matières en suspension.

Le décanteur primaire 100 comporte un conduit d'évacuation 109 des effluents décantés connecté à l'entrée des effluents à filtrer d'un filtre 1 ou d'une installation de filtration 34 comportant plusieurs filtres 1 fonctionnant en parallèle, via un conduit d'alimentation 4, et des moyens d'évacuation des boues issues de la décantation.

Les boues issues de la décantation primaire sont évacuées vers le digesteur, par des moyens d'évacuation, tels qu'un conduit d'évacuation 111, connectés au conduit d'alimentation 207 en boues fraîches du digesteur 200.

Le conduit d'évacuation 209 des effluents est connecté à l'entrée d'alimentation en effluents à décanter du décanteur primaire 100. La sortie d'évacuation des boues digérées est connectée, via le conduit d'évacuation 212, à l'entrée d'un épaississeur 48.

L'épaississeur 48 comporte une sortie 49 de l'eau résiduelle connectée à l'entrée d'alimentation en effluents à décanter du décanteur primaire 100, éventuellement via le conduit d'évacuation 209 des effluents du digesteur 200.

L'épaississeur 48 comporte également une sortie 50 des boues épaissies. Ces boues peuvent subir un conditionnement et un traitement appropriés en vue de leur utilisation, notamment pour l'amendement des sols. Elles peuvent également être dirigées vers une décharge ou une unité d'incinération.

Les effluents filtrés par le filtre 1, ou l'installation de filtration 34, sont évacués par un conduit d'évacuation 15. Dans le cas où le filtre 1 est apte à retenir les matières en suspension contenues dans les effluents à filtrer, les effluents filtrés sont rejetés directement. Dans le cas inverse, les effluents filtrés sont dirigés vers l'entrée d'un décanteur secondaire 51.

Le filtre 1 comprend également des sorties de soutirage permettant l'évacuation de la biomasse par des conduits de soutirage 24, ceux-ci pouvant être connectés soit à l'entrée du décanteur secondaire 51, soit, lorsque la station ne comprend pas de décanteur secondaire, à l'entrée d'alimentation en effluents à décanter du décanteur primaire 100 ou à l'entrée d'alimentation en boues fraîches du digesteur 200.

Le décanteur secondaire 51 comporte des moyens d'évacuation des boues issues de la décantation, connectés soit à l'entrée d'alimentation en effluents à décanter du décanteur primaire 100, via un conduit d'évacuation 52, débouchant éventuellement dans le conduit 209, soit à l'entrée d'alimentation en boues fraîches du digesteur 200. Une partie de ces boues peut également être renvoyée en tête du filtre 1 pour en maintenir la biomasse à un niveau désiré.

En outre, le décanteur secondaire 51 comporte un conduit d'évacuation 53 des effluents décantés.

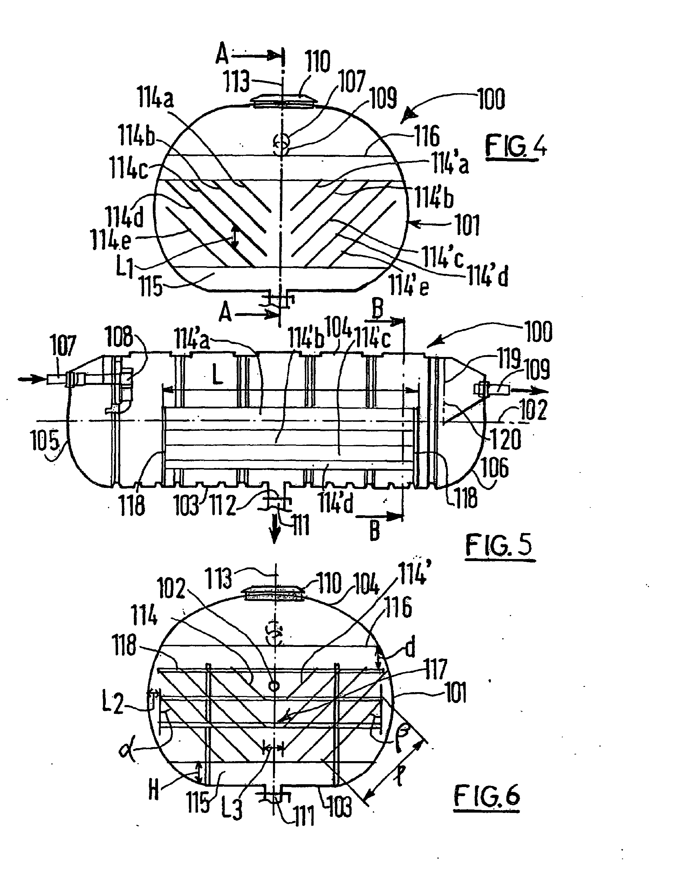

Dans une réalisation possible, représentée sur les figures 4 à 6, le décanteur primaire 100 et/ou le décanteur secondaire 51 comprend, selon une définition générale :
- une cuve 101 ayant un fond 103 et présentant, par rapport au sens de l'écoulement des effluents, une partie amont dans laquelle débouche un conduit d'alimentation 107 en effluents usés et une partie aval dans laquelle débouche le conduit d'évacuation 109 des effluents décantés ;
- une surface de décantation disposée dans la cuve 101, formée par la face supérieure d'au moins un panneau 114, 114' de décantation, ledit panneau présentant un plan moyen sensiblement parallèle au sens d'écoulement des effluents et incliné, dans un plan transversal à l'écoulement des effluents, et par rapport à la projection orthogonale de la verticale dans ledit plan transversal, d'un angle (α, β).

Un premier ensemble d'au moins un panneau de décantation est incliné selon un premier angle (α) compris entre 15° et 60°, et au moins un deuxième ensemble d'au moins un panneau de décantation est incliné selon un deuxième angle (β) compris entre 15° et 60°, les angles (α, β), l'état de surface et le coefficient de frottement des panneaux étant choisis de sorte que, lors de l'écoulement des effluents dans la cuve, les boues se déposent sur la surface de décantation puis glissent vers le fond de la cuve, au moins un passage d'évacuation 117 des boues étant prévu entre les panneaux des deux ensembles, de sorte à permettre aux boues collectées sur les faces supérieures des panneaux de tomber par gravité sur le fond 103 de la cuve 101.

La cuve 101 est sensiblement cylindrique, enterrée de sorte que l'axe 102 soit horizontal, et fermée à l'exception de quatre ouvertures :
- une ouverture d'entrée des effluents à décanter, ménagée dans la paroi d'extrémité amont 105, le conduit d'alimentation 107 se prolongeant à l'intérieur de la cuve par un coude 108 ouvert vers l'amont ;
- une ouverture d'évacuation des effluents décantés, ménagée dans la paroi d'extrémité aval 106, une paroi siphoïque 119 munie d'une ouverture 120 étant prévue pour retenir les matières flottantes à l'intérieur de la cuve ;
- une trappe de visite 110, dans la paroi supérieure 104 ;
- éventuellement, un orifice d'évacuation des boues issues de la décantation, connecté à un conduit d'évacuation 111, des moyens de coupure 112 étant prévus. En variante, les moyens d'évacuation peuvent comporter des pompes refoulantes immergées dans la cuve 101 ou des tuyaux aspirants remontant au-dessus de la cuve 101 et reliés à une pompe aspirante.
   La cuve comporte deux ensembles de panneaux, disposés symétriquement par rapport au plan 113, vertical, longitudinal et médian de la cuve.

Le premier ensemble (à gauche) comprend cinq panneaux 114a à 114e sensiblement parallèles, superposés verticalement, et écartés les uns des autres d'une distance sensiblement constante L1 de l'ordre de 30 cm. Les panneaux 114a à 114e sont inclinés de haut en bas depuis la paroi latérale sensiblement verticale de la cuve vers le plan vertical 113, d'un angle α de l'ordre de 45°.

Les panneaux 114 présentent des largeurs I différentes selon leur distance par rapport au fond 103 de la cuve, mais des longueurs L (parallèlement à l'écoulement des effluents) sensiblement égales. Les panneaux 114 sont écartés de la paroi latérale verticale de la cuve 101 d'une distance horizontale L2 de l'ordre de 10 cm, pour le passage des matières en suspension, et du fond 103 d'une hauteur verticale H voisine de 30 cm, pour ménager un espace 115 d'accumulation des boues issues de la décantation. Enfin, l'extrémité supérieure des panneaux est située à une distance d au-dessous du niveau 116 des effluents à l'intérieur de la cuve.

Le deuxième ensemble (à droite) est symétrique du premier par rapport au plan vertical 113, et comprend cinq panneaux 114'a à 114'e. L'écartement horizontal L3 entre les premier et deuxième ensembles de panneaux est voisin de 30 cm, de sorte à ménager un espace 117 permettant aux boues collectées sur les panneaux de tomber vers le fond en étant dirigées et recueillies en zone centrale du fond 103 de la cuve 101.

Des moyens 118 de support et de fixation des panneaux 114, 114' à la cuve 101 sont disposés au voisinage des extrémités amont et aval desdits panneaux.

Selon une réalisation possible, le décanteur 100 comprend, par rapport au sens de l'écoulement des effluents, au moins une première et une deuxième série de panneaux 114, 114', la deuxième série étant située en aval de la première série, lesdites séries comportant chacune au moins un premier et un deuxième ensembles d'au moins un panneau, de sorte à améliorer encore l'extraction des boues.

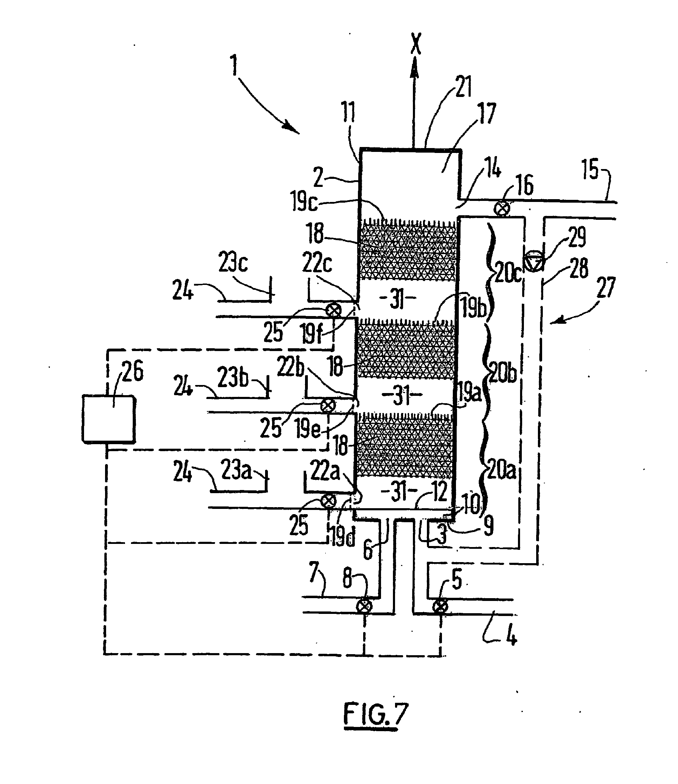

Dans une réalisation possible, représentée sur la figure 7, le filtre 1 comprend, selon une définition générale, un réacteur 2 dans lequel le flux d'effluents à filtrer est prévu pour circuler de bas en haut, ledit réacteur 2 comprenant :
- en partie basse, une entrée 3 des effluents à filtrer et une entrée 6 de gaz oxygéné ;
- et, en partie haute, une sortie 14 des effluents filtrés ;
- des moyens filtrants 18 comprenant des couches de particules d'un matériau solide formant supports et une biomasse accrochée sur la surface desdits supports, lesdits moyens filtrants 18 ayant une densité inférieure à la densité des effluents à filtrer et étant interposés entre l'entrée 3 des effluents à filtrer et la sortie 14 des effluents filtrés.

Le réacteur 2 est subdivisé en au moins trois compartiments superposés formant étages au moyen d'au moins deux parois 19a, 19b, 19c munies d'ouvertures, lesdites ouvertures étant agencées pour retenir les moyens filtrants 18, de sorte à former dans le réacteur :
- au moins deux étages de filtration 20a, 20b, 20c ;
- et un étage supérieur de sortie 17, la sortie 14 des effluents filtrés débouchant dans l'étage supérieur de sortie 17.
   Chaque étage de filtration 20a, 20b, 20c est pourvu d'une couche de ses propres moyens filtrants 18 et comprend, en partie inférieure, une sortie de soutirage 22a, 22b, 22c de la biomasse excédentaire, la quantité et la densité des moyens filtrants 18 dans chaque étage de filtration 20a, 20b, 20c étant telles que, en mode soutirage, la partie inférieure d'au moins le ou les étages inférieurs 20a, 20b dans laquelle débouche la sortie de soutirage 22a, 22b est exempte de moyens filtrants, de manière à permettre la récupération de la biomasse excédentaire.

Le réacteur 2 définit un axe principal X orienté verticalement. L'entrée 3 des effluents à filtrer est reliée à un conduit d'alimentation 4 muni d'une vanne 5, et l'entrée 6 de gaz oxygéné est reliée à un conduit d'alimentation 7 muni d'une vanne 8. Selon un mode de réalisation, les deux entrées 3, 6 sont prévues dans la paroi inférieure 9 du réacteur 2 et débouchent dans un compartiment d'entrée 10 délimité par une partie de la paroi latérale 11 du réacteur, et par une paroi supérieure interne 12 perméable aux effluents et au gaz oxygéné.

La sortie 14 des effluents filtrés est reliée à un conduit d'évacuation 15 muni d'une vanne 16, et débouche dans un étage supérieur de sortie 17 du réacteur.

Le réacteur 2 est subdivisé en compartiments superposés formant étages de filtration, par des grilles 19a, 19b, 19c, l'étage supérieur de sortie 17, formé entre la paroi supérieure 21 du réacteur et la paroi 19c supérieure disposée en regard, étant exempt de moyens filtrants.

Chaque étage de filtration 20a, 20b, 20c comprend en partie inférieure une sortie de soutirage 22a, 22b, 22c de la biomasse excédentaire, connectée à un conduit de soutirage 24 muni d'une vanne 25. Un compartiment de récupération 23a, 23b, 23c de ladite biomasse excédentaire peut être prévu.

Les moyens filtrants 18 sont prévus pour flotter, au moins dans certains compartiments, en mode soutirage, de sorte à former un espace inférieur 31 libre exempt de moyens filtrants. Des grilles 19d, 19e, 19f permettent de retenir les moyens filtrants 18. Un moyen de détection 26 de la quantité de biomasse excédentaire peut également être prévu.

Le filtre 1 peut comprendre des moyens de recirculation 27 des effluents filtrés, comportant un conduit 28 associé à une pompe 29, pour traitement supplémentaire d'au moins une partie des effluents filtrés.

Lors de la phase de filtration, les effluents et le gaz circulent de façon ascendante à travers les différents étages de filtration. Les effluents filtrés sont évacués par le conduit 15, et la biomasse reste sur les supports. L'alimentation en effluents et/ou en gaz est continue ou discontinue.

Lors de la phase de lavage, la biomasse excédentaire de l'étage choisi est récupéré par le conduit de soutirage 24 correspondant par flux descendant des effluents contenus dans le réacteur 2.

## Revendications

1. Procédé de digestion anaérobie de boues issues du traitement des effluents usés, **caractérisé en ce qu'**il comprend les étapes consistant à :
- introduire dans la partie amont d'un digesteur (200) anaérobie de boues un flux de boues fraîches, lesdites boues fraîches contenant des effluents et présentant une concentration en matière sèche inférieure à 40 g/l ;
- maintenir dans le digesteur (200) une couche de boues d'épaisseur inférieure à 0,6m, les boues étant transformées par digestion anaérobie ;
- faire circuler lesdits effluents dans le digesteur (200), au-dessus et à travers la couche de boues, de façon sensiblement horizontale au moins dans la partie utile du digesteur, de la partie amont vers la partie aval du digesteur, et évacuer lesdits effluents hors du digesteur (200), de sorte à évacuer les produits solubles de la digestion des boues hors du digesteur ;
- évacuer les boues hors du digesteur (200).

2. Procédé selon la revendication 1, **caractérisé en ce qu'**on maintient dans le digesteur (200) une couche de boues d'une épaisseur inférieure à 0,5 m.

3. Procédé selon la revendication 1 ou 2, **caractérisé en ce que** les boues fraîches présentent une concentration en matière sèche inférieure à 20 g/l.

4. Procédé selon l'une quelconque des revendications 1 à 3, **caractérisé en ce que** les boues fraîches introduites dans le digesteur sont obtenues via une dilution de boues ayant subi une opération préalable d'épaississement par des effluents de dilution.

5. Procédé selon la revendication 4, **caractérisé en ce que** les effluents de dilution sont des effluents issus de la décantation d'effluents usés.

6. Procédé selon l'une quelconque des revendications 1 à 5, **caractérisé en ce qu'**on chauffe le digesteur (200) à une température comprise entre 20 et 35°C.

7. Procédé selon l'une quelconque des revendications 1 à 6, **caractérisé en ce qu'**on évacue les boues digérées par vidange périodique du digesteur (200).

8. Procédé selon l'une quelconque des revendications 1 à 6, **caractérisé en ce qu'**on évacue les boues digérées par soutirage périodique ou continu en partie inférieure et avale du digesteur (200).

9. Digesteur anaérobie de boues fraîches contenant des effluents, pour la mise en oeuvre du procédé selon l'une des revendications précédentes, le digesteur (200) comprenant une cuve (201) ayant un fond (203) sensiblement horizontal, un conduit d'alimentation (207) de la cuve en boues fraîches, un conduit d'évacuation (209) des effluents hors de la cuve, et des moyens d'évacuation (212) des boues digérées hors de la cuve, ladite cuve comportant au moins une paroi (213a, 213b, 213c) transversale à l'écoulement des effluents, la paroi définissant un compartiment amont (218a, 218b, 218c) et un compartiment aval (218b, 218c, 218d), de sorte que la cuve présente un premier compartiment amont (218a), dans lequel débouche le conduit d'alimentation (207) en boues fraîches, et un dernier compartiment aval (218d), dans lequel débouche le conduit d'évacuation (209) des effluents, **caractérisé en ce que** la paroi (213a, 213b, 213c) présente, à sa partie inférieure, une ouverture de communication (216) du compartiment amont au compartiment aval, de sorte à permettre le passage des boues et la circulation des effluents, au-dessus et à travers la couche de boues maintenue au fond de la cuve, de façon sensiblement horizontale du premier compartiment amont (218a) au dernier compartiment aval (218d).

10. Digesteur selon la revendication 9, **caractérisé en ce que** la paroi transversale (213a, 213b, 213c) s'étend depuis le fond (203) de la cuve sur une hauteur inférieure à la hauteur de la cuve (201), de sorte à permettre le débordement des matières flottantes des effluents d'un compartiment (218a, 218b, 218c, 218d) à un autre.

11. Digesteur selon la revendication 10, **caractérisé en ce que** la paroi transversale (213a, 213b, 213c) s'étend depuis le fond (203) de la cuve sur une hauteur comprise entre 40 et 70% de la hauteur de la cuve (201).

12. Digesteur selon l'une des revendications 9 à 11, **caractérisé en ce que** l'ouverture de communication (216) s'étend depuis le fond (203) de la cuve (201) sur une hauteur comprise entre 30 et 60% de la hauteur de la paroi transversale (213a, 213b, 213c).

13. Digesteur selon l'une des revendications 9 à 12, **caractérisé en ce que** l'ouverture de communication (216) présente un bord supérieur (217) sensiblement annulaire.

14. Digesteur selon l'une des revendications 9 à 13, **caractérisé en ce que** la paroi transversale (213a, 213b, 213c) s'étend sur toute la largeur de la cuve.

15. Digesteur selon l'une des revendications 9 à 14, **caractérisé en ce que** la cuve (201) est sensiblement cylindrique et d'axe (202) sensiblement horizontal.

16. Digesteur selon l'une des revendications 9 à 15, **caractérisé en ce qu'**il comporte au moins deux parois transversales (213a, 213b, 213c) présentant chacune, à leur partie inférieure, une ouverture de communication (216), de sorte à définir au moins trois compartiments (218a, 218b, 218c, 218d) successifs dans la cuve (201).

17. Digesteur selon l'une des revendications 1 à 16, **caractérisé en ce que** le conduit d'alimentation (207) débouche en partie supérieure du premier compartiment amont (218a) de la cuve (201 ).

18. Digesteur selon l'une des revendications 1 à 17, **caractérisé en ce que** le conduit d'évacuation (209) comporte une prise siphoïque (210) disposée sensiblement à mi hauteur entre le fond (203) de la cuve et le niveau des effluents (219) dans ladite cuve (201).

19. Digesteur selon l'une des revendications 1 à 18, **caractérisé en ce que** les moyens d'évacuation des boues digérées comprennent un orifice d'évacuation ménagé dans le fond (203) de la cuve (201) et un conduit d'évacuation (212) desdites boues connecté audit orifice d'évacuation.

20. Station d'épuration d'effluents usés, **caractérisée en ce qu'**elle comprend au moins un digesteur (200) selon l'une des revendications 1 à 19, ladite station comprenant en outre un décanteur primaire (100) alimenté en effluents usés, ledit décanteur primaire (100) comportant des moyens d'évacuation (111) des boues issues de la décantation connectés au conduit d'alimentation (207) du digesteur (200) en boues fraîches, et un conduit d'évacuation (109) des effluents décantés.

21. Station d'épuration selon la revendication 20, **caractérisée en ce que** le décanteur primaire (100) comprend :
- une cuve (101) ayant un fond (103) et présentant, par rapport au sens de l'écoulement des effluents, une partie amont dans laquelle débouche un conduit d'alimentation (107) en effluents usés et une partie aval dans laquelle débouche le conduit d'évacuation (109) des effluents décantés ;
- une surface de décantation disposée dans la cuve (101), formée par la face supérieure d'au moins un panneau (114, 114') de décantation, ledit panneau présentant un plan moyen sensiblement parallèle au sens d'écoulement des effluents et incliné, dans un plan transversal à l'écoulement des effluents, et par rapport à la projection orthogonale de la verticale dans ledit plan transversal, d'un angle (α, β) ; un premier ensemble d'au moins un panneau (114, 114a, 114b, 114c, 114d, 114e) de décantation étant incliné selon un premier angle (α) compris entre 15° et 60°, et au moins un deuxième ensemble d'au moins un panneau (114', 114'a, 114'b, 114'c, 114'd, 114'e) de décantation étant incliné selon un deuxième angle (β) compris entre 15° et 60°, les angles (α, β), l'état de surface et le coefficient de frottement des panneaux étant choisis de sorte que, lors de l'écoulement des effluents dans la cuve, les boues se déposent sur la surface de décantation puis glissent vers le fond de la cuve, au moins un passage d'évacuation (117) des boues étant prévu entre les panneaux des deux ensembles, de sorte à permettre aux boues collectées sur les faces supérieures des panneaux de tomber par gravité sur le fond (103) de la cuve (101).

22. Station d'épuration selon la revendication 20 ou 21, **caractérisée en ce qu'**elle comprend en outre un filtre (1) comportant :
- une entrée (3) en effluents à filtrer, connectée au conduit d'évacuation (109) des effluents décantés ;
- une sortie (14) des effluents filtrés ;
- une sortie de soutirage (22a, 22b, 22c) de la biomasse excédentaire.

23. Station d'épuration selon la revendication 22, **caractérisée en ce que** le filtre comprend un réacteur (2) dans lequel le flux d'effluents à filtrer est prévu pour circuler de bas en haut, ledit réacteur (2) comprenant :
- en partie basse, une entrée (3) des effluents à filtrer et une entrée (6) de gaz oxygéné ;
- et, en partie haute, une sortie (14) des effluents filtrés ;
- des moyens filtrants (18) comprenant des couches de particules d'un matériau solide formant supports et une biomasse accrochée sur la surface desdits supports, lesdits moyens filtrants (18) ayant une densité inférieure à la densité des effluents à filtrer et étant interposés entre l'entrée (3) des effluents à filtrer et la sortie (14) des effluents filtrés ; le réacteur (2) étant subdivisé en au moins trois compartiments superposés formant étages au moyen d'au moins deux parois (19a, 19b, 19c) munies d'ouvertures, lesdites ouvertures étant agencées pour retenir les moyens filtrants (18), de sorte à former dans le réacteur (2) :
- au moins deux étages de filtration (20a, 20b, 20c) ;
- et un étage supérieur de sortie (17), la sortie (14) des effluents filtrés débouchant dans l'étage supérieur de sortie (17) ; chaque étage de filtration (20a, 20b, 20c) étant pourvu d'une couche de ses propres moyens filtrants (18), et comprenant, en partie inférieure, une sortie de soutirage (22a, 22b, 22c) de la biomasse excédentaire, la quantité et la densité des moyens filtrants (18) dans chaque étage de filtration (20a, 20b, 20c) étant telles que, en mode soutirage, la partie inférieure d'au moins le ou les étages inférieurs (20a, 20b) dans laquelle débouche la sortie de soutirage (22a, 22b) est exempte de moyens filtrants, de manière à permettre la récupération de la biomasse excédentaire.

24. Station d'épuration selon la revendication 22 ou 23, **caractérisée en ce qu'**elle comprend en outre un décanteur secondaire (51 ) comportant :
- une entrée des effluents à décanter connectée aux sorties de soutirage (22a, 22b, 22c) du filtre (1 ) ;
- une sortie des effluents décantés ;
- des moyens d'évacuation des boues issues de la décantation.

25. Station d'épuration selon l'une des revendications 20 à 24, **caractérisée en ce qu'**elle comprend en outre un épaississeur (48) comprenant :
- une entrée connectée à la sortie d'évacuation des boues digérées du digesteur (200) ;
- une sortie des boues épaissies ;
- une sortie de l'eau résiduelle connectée à l'entrée d'alimentation en effluents à décanter du décanteur primaire (100).
